# EUROPEAN PATENT APPLICATION

(11) **EP 4 458 308 A1**
(43) Date of publication of application: **06.11.2024**
(21) Application number: 22914875.4
(22) Date of filing: 28.12.2022
(51) Int. Cl.: A61B 34/37

(54) **MASTER-SIDE CONTROL MECHANISM AND SURGICAL ROBOT**

(30) Priority: 31.12.2021 CN 202111671630
(71) Applicant: Zhicheng Medical Technology (Jiaxing) Co., Ltd., Jiaxing, Zhejiang 314400 (CN)
(72) Inventor: WANG, Sixin, Suzhou, Jiangsu 215000 (CN); XU, Weiliang, Suzhou, Jiangsu 215000 (CN); ZHANG, Yi, Suzhou, Jiangsu 215000 (CN)
(74) Representative: Klunker IP Patentanwälte PartG mbB
(86) International application number: PCT/CN2022/142666
(87) International publication number: WO 2023/125623

(57) **Abstract**

A master-side control mechanism and a surgical robot are provided. The master-side control mechanism includes a base, two fixing frames, a rotating shaft rotatably connected to the two fixing frames and provided with a baffle, a first sensing device configured to sense the rotational movement of the rotating shaft, two handles located on both sides of the baffle and sleeved on the rotating shaft and capable of moving opposite to each other along the axial direction thereof, two second sensing devices configured to sense axial movements of the two handles corresponding to an advancing action and a retracting action and a control device. The control device is configured to control the slave-side execution apparatus to perform rotating and twisting actions based on the rotational movement of the rotating shaft and to perform the advancing or retracting action based on the axial movement of the handles .

## Description

### CROSS REFERENCE

The present application refers to the Chinese patent application No. 202111671630.8 entitled "MASTER-SIDE CONTROL MECHANISM AND SURGICAL ROBOT" filed on December 31, 2021, which is incorporated by reference into this application in its entirety.

### TECHNICAL FIELD

The present disclosure relates to the technical field of medical instruments, in particular to a master-side control mechanism and a surgical robot.

### BACKGROUND TECHNIQUE

At present, vascular interventional surgery guided by digital subtraction angiography (DSA) requires doctors to be exposed to X-rays throughout the whole process. A large amount of radiation for a long time will lead to leukopenia, low immunity and other conditions, which seriously threatens the health of doctors. Moreover, wearing radiation protective clothing will consume the doctor's physical strength, which greatly affects the accuracy of the surgery. Therefore, in recent years, interventional surgical robots have gradually emerged. Doctors can remotely manipulate the master-side manipulating handle of the robot to control the advancing and retracting and rotational actions of a guidewire or catheter at the tail end of the robot in an operating room, thereby avoiding ray radiation and improving surgical accuracy.

The master-side manipulating device of the interventional surgical robot is the part directly contacted by the doctor's hands. The doctor controls the advancing and retracting and rotational actions of the guidewire or catheter by pushing, pulling, rotating and twisting the master-side manipulating handle, whereas in the past, the doctor operated the guidewire or catheter by pinching it directly with the thumb and forefinger, so it is inevitable that there are differences between the two. In other words, the operation feel of the interventional surgical robot is different from that of the guidewire or catheter, which leads to a decrease in the doctor's sense of presence during an actual surgical process and limits the use of the doctor's experience.

Therefore, a master-side control mechanism (may also be translated as manipulating apparatus) and a surgical robot are needed to at least partially solve the above problems.

### SUMMARY

A series of simplified concepts is introduced into the portion of SUMMARY, which would be further described in detail in the portion of DETAILED DESCRIPTION. The portion of SUMMARY of the present disclosure does not mean attempting to define the key features and essential technical features of the technical solution claimed to be protected, let alone determining the protection scope of the technical solution claimed to be protected.

To at least partially solve the problems, a first aspect of the present disclosure provides a master-side control mechanism used for a surgical robot, comprising:
a base;
two fixing frames disposed opposite to each other at both ends of the base;
a rotating shaft rotatably connected to the two fixing frames and provided with a baffle in the middle;
a first sensing device configured to sense a rotational movement of the rotating shaft;
two handles respectively located on both sides of the baffle and sleeved on the rotating shaft, the two handles being configured to be capable of moving opposite to each other relative to the rotating shaft along an axial direction of the rotating shaft;
two second sensing devices configured to respectively sense an axial movement of the two handles relative to the rotating shaft; and
a control device in signal connection with the first sensing device and the two second sensing devices;
wherein the control device is configured to control a slave-side execution apparatus of the surgical robot to perform rotating and twisting actions based on the rotational movement of the rotating shaft sensed by the first sensing device; and
the axial movement of the two handles corresponds respectively to an advancing action and a retracting action of the slave-side execution apparatus, and the control device is configured to control the slave-side execution apparatus to perform the advancing action or the retracting action based on the axial movement of the two handles sensed by the two second sensing devices.

The master-side control mechanism according to the present disclosure is capable of simultaneously simulating the pushing, pulling, rotating and twisting actions of the guidewire or catheter, and the two handles are disposed relatively independently, so that a non-interfering interlocking between the advancing action and the retracting action can be realized while the operation feel of pinching the guidewire or catheter is simulated, and the speed of the pushing and pulling actions can be adjusted.

A second aspect of the present disclosure provides a surgical robot comprising the master-side control mechanism described in the first aspect.

The surgical robot according to the present disclosure can achieve similar technical effects to the master-side control mechanism described in the first aspect.

### BRIEF DESCRIPTION OF THE DRAWINGS

The following drawings are hereby incorporated as part of the present disclosure for the understanding of the present disclosure. The embodiments are illustrated and described in the drawings in order to explain the principles of the present disclosure.

In the drawings:
FIG. 1 is a schematic view of a master-side control mechanism according to an embodiment of the present disclosure;
FIG. 2 is a schematic sectional view of the master-side control mechanism in FIG. 1;
FIG. 3 is a schematic sectional view of an operating device of the master-side control mechanism according to an embodiment of the present disclosure;
FIG. 4 is an enlarged schematic view of part A in FIG. 4;
FIG. 5 is a schematic view of a base assembly and a speed regulating device of the master-side control mechanism according to an embodiment of the present disclosure;
FIG. 6 is a schematic sectional view of the base assembly and the speed regulating device in FIG. 5;
FIG. 7 is an enlarged schematic view of part B in FIG. 6;
FIG. 8 is a schematic view of the master-side control mechanism according to another embodiment of the present disclosure;
FIG. 9 is a schematic sectional view of the master-side control mechanism in FIG. 8; and
FIG. 10 is a partial schematic view of a radial section along a rotating shaft of the master-side control mechanism according to an embodiment of the present disclosure.

### DETAILED DESCRIPTION

In the following description, numerous specific details are set forth in order to provide a more thorough understanding of the present disclosure. However, it is obvious to those skilled in the art that the present disclosure may be implemented without one or more of these details. Some technical features well-known in the art are not described in other examples in order to avoid confusion with the present disclosure.

In order to thoroughly understand the present disclosure, a detailed description will be provided in the following description. It should be understood that these embodiments are provided so that the disclosure of the present disclosure will be thorough and complete and the concepts of these exemplary embodiments will be completely delivered to those ordinary skilled in the art. Obviously, the implementation of the present disclosure is not limited to the specific details familiar to those skilled in the art. The preferred embodiments of the present disclosure are described in detail as follows. However, in addition to these detailed descriptions, the present disclosure may have other embodiments.

It shall be noted that the terminology used herein is for the purpose of describing particular embodiments only and is not intended to limit the exemplary embodiments in accordance with the present disclosure. As used herein, unless the context clearly indicates, otherwise the singular forms are intended to include the plural forms as well. It will be further understood that the terms "comprising" and/or "including" and their derivatives as used herein, specify the presence of stated features, wholes, steps, operations, elements, and/or components, but do not exclude the presence or addition of one or more other features, wholes, steps, operations, elements, components, and/or combinations thereof.

Ordinals such as "first" and "second" recited in the present disclosure are merely identifiers and do not have any other meaning, for example, a particular order and the like. Moreover, for example, the term "first component" itself does not imply an existence of "second component", and the term "second component" itself does not imply an existence of "first component." It should be noted that the terms "up", "down", "front", "back", "left", "right", "inside", "outside" and similar expressions used herein are for illustrative purposes only and are not restrictive.

A surgical robot (not shown) in an embodiment of the present disclosure includes a master-side control mechanism 100 and a slave-side execution apparatus (not shown). Wherein the slave-side execution apparatus is used to perform operations such as feeding or withdrawing a guidewire or catheter to a patient in an operating room. The master-side control mechanism 100 is used for operation by doctors outside the operating room. The doctor controls the action of the slave-side execution apparatus by operating the master-side control mechanism 100.

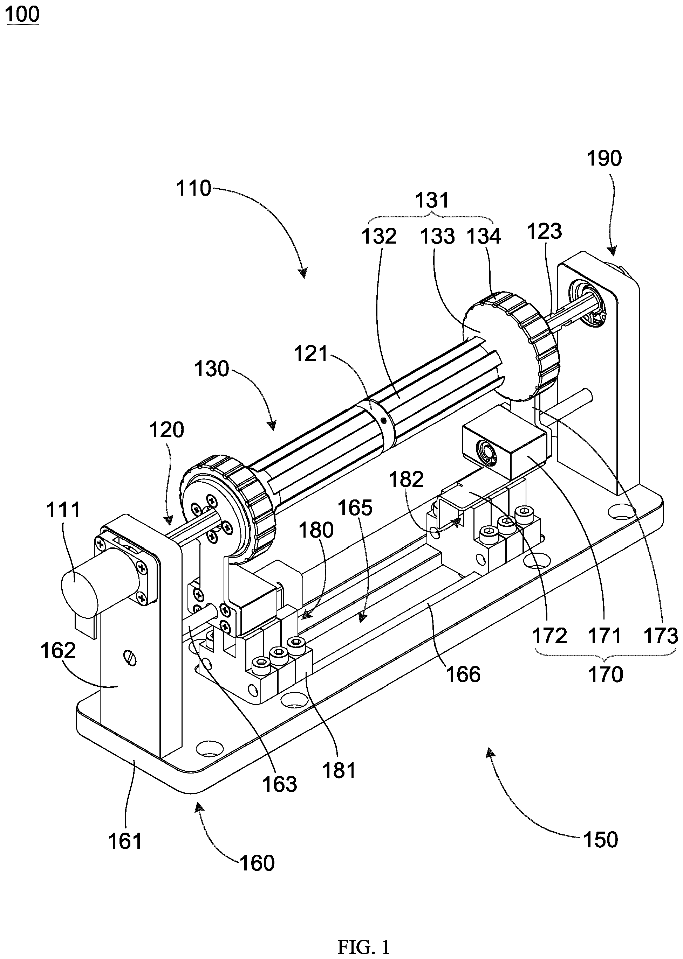

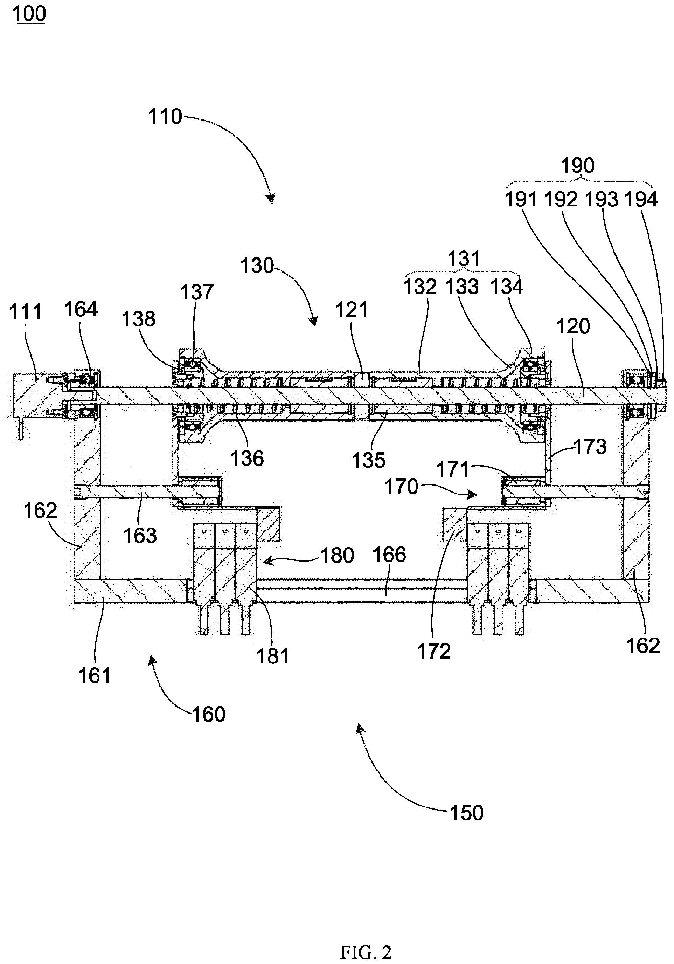

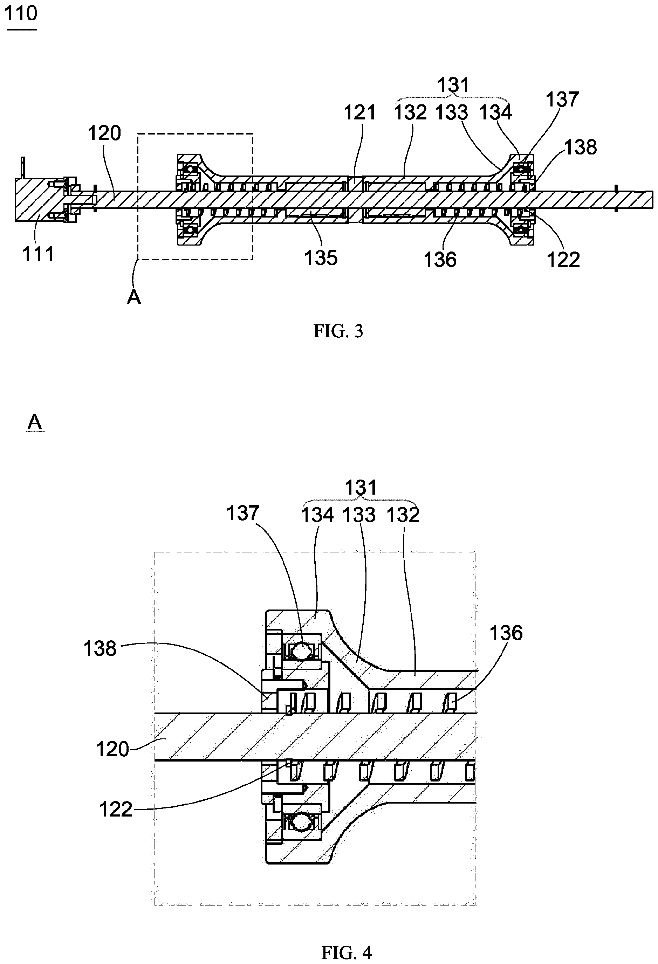

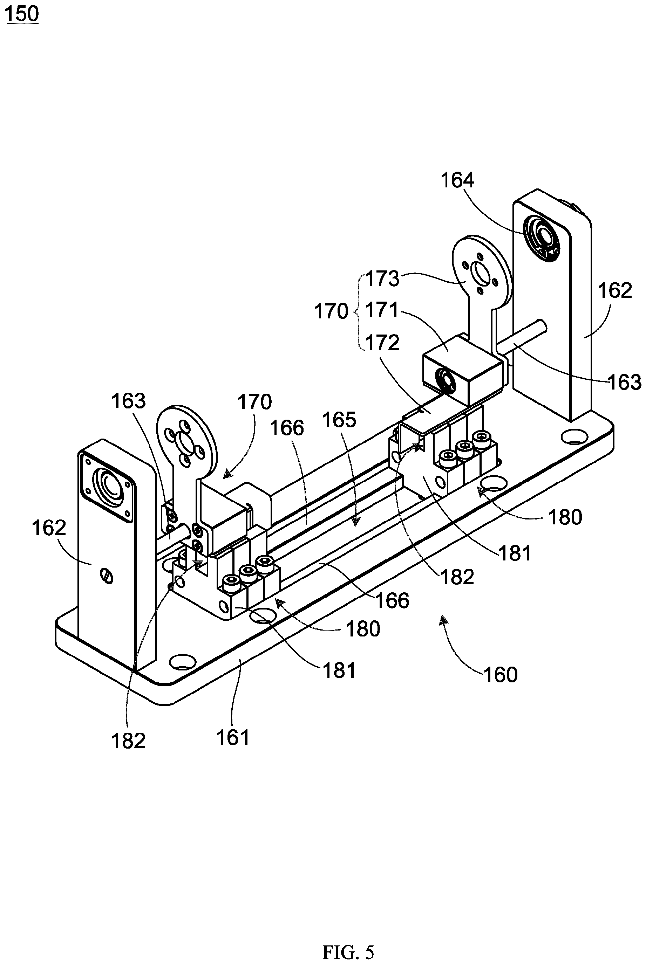

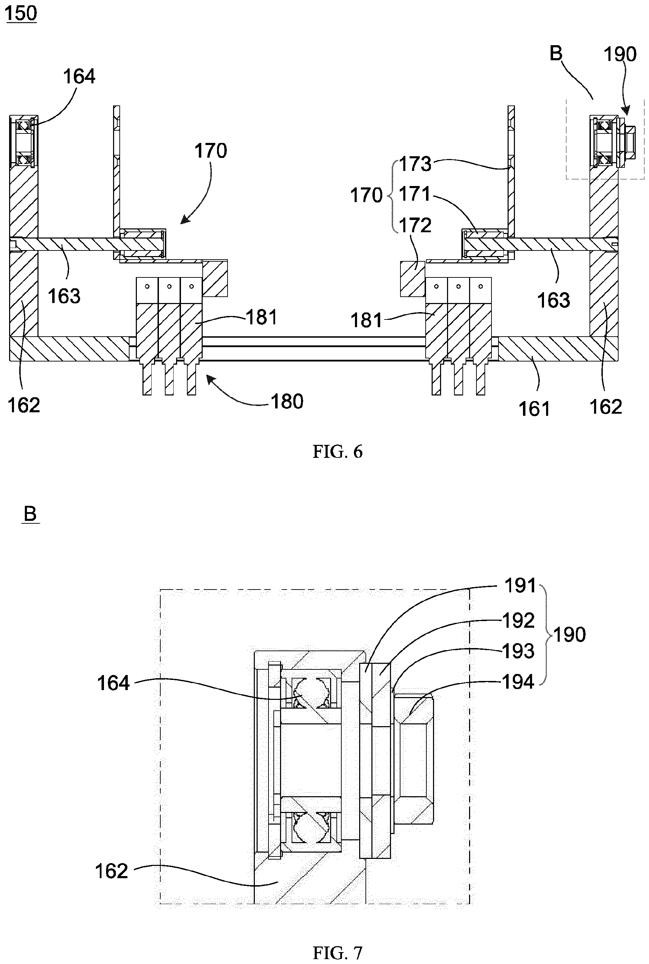

The master-side control mechanism is described in more detail with reference to FIGS. 1 to 7. The master-side control mechanism 100 in an embodiment of the present disclosure includes a base assembly 160, an operating device 110, a speed regulating device 150 and a control device (not shown). The operating device 110 and the speed regulating device 150 are both disposed on the base assembly 160. Wherein the base assembly 160 includes a base 161 and a fixing frame 162. Optionally, two fixing frames 162 are disposed opposite to each other at both ends of the base 161.

Referring first to FIGS. 1, 2, 3 and 4, the operating device 110 includes a rotating shaft 120 rotatably disposed on the two fixing frames 162, and two handles 130 sleeved on the rotating shaft 120 and configured to be capable of moving opposite to each other relative to the rotating shaft 120 in an axial direction thereof. Wherein the two handles 130 correspond to the advancing action and the retracting action of the salve-side execution apparatus, respectively (described in more detail below). Optionally, the rotating shaft 120 is provided with a baffle 121 located in the middle of the rotating shaft 120 to separate the two handles 130, such that the two handles 130 can be relatively independent of each other to avoid interference therebetween, and also such that the other action will not be accidentally triggered when one of the advancing and the retracting actions is operated by a doctor, so as to realize interlocking. Moreover, it may be possible to allow the doctor to operate the other handle 130 in passing as one of the handles 130 is reset when switching the advancing and retracting actions, so as to realize seamless switching.

Specifically, the handle 130 includes an operating body 131 in which a conforming portion 135 is disposed, and the operating body 131 is fixedly connected to the conforming portion 135. The conforming portion 135 is configured to be sleeved on the rotating shaft 120 and profile-fit with the rotating shaft 120, such that the operating body 131 is capable of co-rotating with the rotating shaft 120 and is movable along the extension direction of the rotating shaft 120.

The rotating shaft 120 is provided with a spline 123 extending along the axial direction. A ball spline pair 135 is configured to cooperate with the spline 123 such that the operating body 131 can co-rotate with the rotation shaft 120 and move along the extension direction of the spline 123. As a result, the friction between the handle 130 and the rotating shaft 120 is reduced, so that the axial movement is smoother.

The rotating shaft 120 is provided with a blocking portion 122 protruding from the rotating shaft 120. The handle 130 further includes an elastic member 136 disposed between the blocking portion 122 and the conforming portion 135, and the three are preferably fixedly connected. The elastic member 136 is preferably configured as a compression spring to provide an elastic force to the handle 130 to make it tend to move in a direction close to the baffle 121. Accordingly, the handle 130 can be automatically reset when the doctor releases his or her hands during operation of the handle 130. Moreover, the elastic coefficient of the compression spring is preferably such that the doctor will not feel great resistance during pushing and pulling the handle 130. For example, the elastic coefficient of the compression spring may be 0.29 to 0.5 N/mm.

In the embodiment shown in FIGS. 1-9, the conforming portion 135 is configured as a ball spline pair 135, and the rotating shaft 120 is provided with a spline 123 extending in the axial direction. The ball spline pair 135 is configured to cooperate with the spline 123 such that the operating body 131 can co-rotate with the rotating shaft 120 and move along the extension direction of the spline 123. As a result, the friction between the handle 130 and the rotating shaft 120 is reduced, so that the axial movement is smoother.

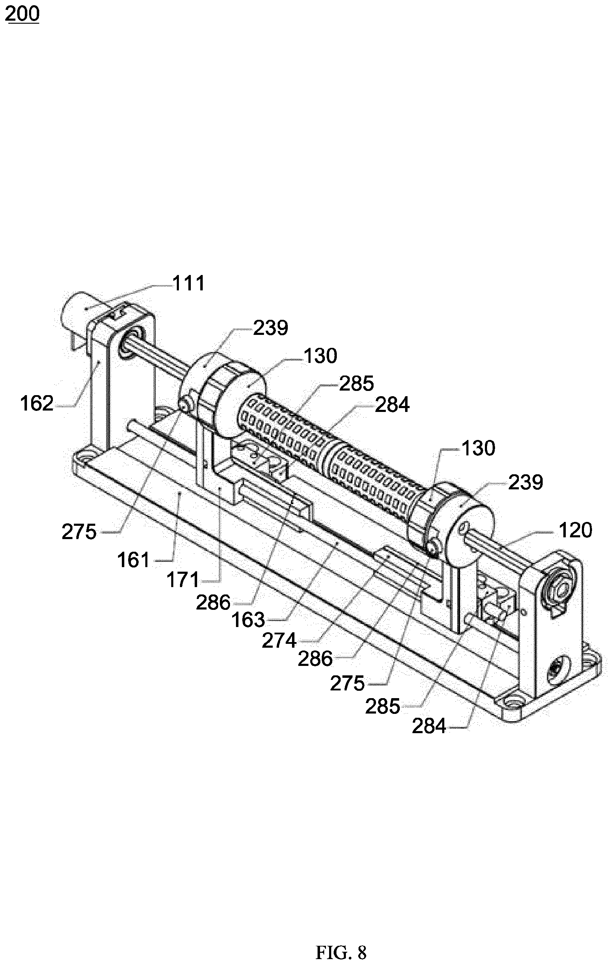

In another optional embodiment, referring to FIGS. 8 to 10, the cross section of the rotating shaft 120 is configured as a rectangle, the conforming portion 135 is provided with a through hole adapted to the rectangle, and the rotating shaft 120 passes through the operating body 131 via the through hole. As a result, the operating body 131 can co-rotate with the rotating shaft 120 through a profile-fit of the through hole with the rectangular cross section, and the rotating shaft 120 passes through the through hole such that the operating body 131 is slidable relative to the rotating shaft 120.

In a preferred embodiment, the conforming portion 135 is disposed at an end of the handle 130 close to the baffle 121. An end of the handle 130 away from the baffle 121 is provided with a first bearing 137, the outer ring of which is connected to the operating body 131, and the inner ring of which is connected to a stator 138. Thereby, the stator 138 is capable of following the movement of the operating body 131 in the axial direction without being affected by the rotational movement of the operating body 131. Wherein the stator 138 is connected with the speed regulating device 150 to transmit the axial movement of the handle 130 to the speed regulating device 150.

As an implementation, the operating body 131 adopts a variable-diameter design. Specifically, the operating body 131 includes a first grip portion 132, a second grip portion 134, and a transition portion 133. Wherein the first grip portion 132 is close to the baffle 121, and the second grip portion 134 is far away from the baffle 121. The transition portion 133 is disposed between the first grip portion 132 and the second grip portion 134. Moreover, the radial dimension of the second grip portion 134 is larger than that of the first grip portion 132. Optionally, the radial dimension of the first grip portion 132 is equal or constant along the axial direction, the radial dimension of the second grip portion 134 is equal or constant along the axial direction, and the radial dimension of the transition portion 133 increases smoothly in the direction from the first grip portion 132 to the second grip portion 134.

In this way, it is possible to facilitate the operation of the doctor while facilitate the arrangement of the first bearing 137 and the stator 138 at the second grip portion 134. Optionally, the outer surface of the operating body 131 is further provided with an anti-skid pattern.

As an optional embodiment, the radial dimension of the operating body 131 may also taper along the direction from its end away from the baffle 121 to its end close to the baffle 121.

In order to facilitate the rotation of the rotating shaft 120, a second bearing 164 is preferably provided on the fixing frame 162 of the base assembly 160. The outer ring of the second bearing 164 is connected to the fixing frame 162, and the inner ring of the second bearing 164 is connected to the rotating shaft 120, so as to enable the rotation to be carried out smoothly. An encoder 111 is provided at an end portion of the rotating shaft 120 to sense a rotation angle of the rotating shaft 120.

In order to better adjust the resistance during rotation and more realistically simulate the rotating and twisting feel of the doctor during operation, a damping assembly 190 is preferably provided. The damping assembly 190 is disposed at an end portion of the rotating shaft 120 passing through the fixing frame 162.

Wherein, the damping assembly 190 includes a friction plate 191, a stop washer 192, a spring washer 193 and a locknut 194. Specifically, the friction plate 191, the stop washer 192, the spring washer 193 and the locknut 194 are all sleeved on the rotating shaft 120. Moreover, the friction plate 191, the stop washer 192, the spring washer 193 and the locknut 194 are arranged in sequence in the direction away from the fixing frame 162. In other words, the friction plate 191 is located at the innermost side, and the locknut 194 is located at the outermost side.

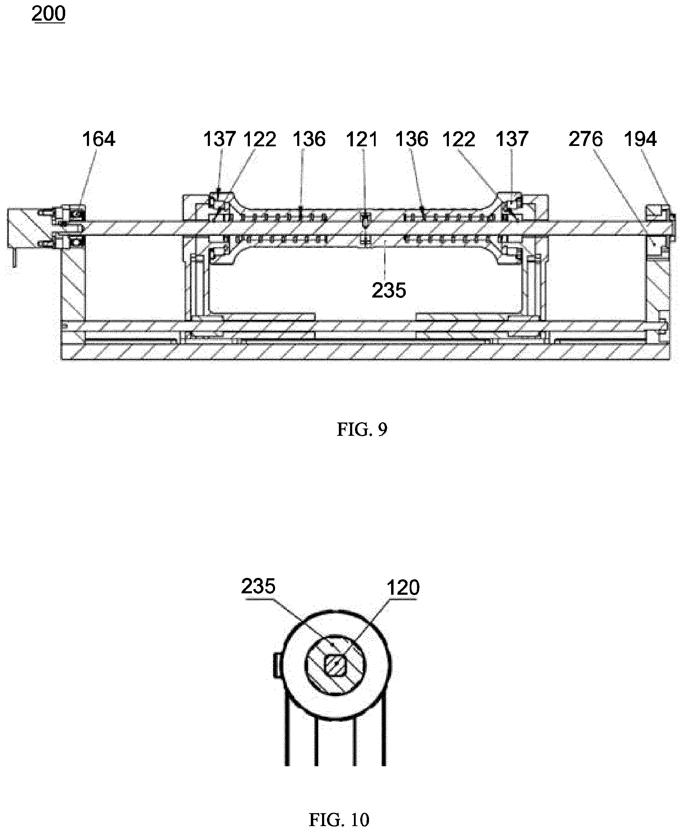

In another optional embodiment, such as the embodiment shown in FIGS. 8 and 9, the damping assembly includes a damping ring 276 sleeved on the rotating shaft 120 and a locknut 194 sleeved on the rotating shaft 120 and connected to a side of the fixing frame 162. The doctor can adjust the rotational damping of the handle 130 by means of the damping ring 276 and the locknut 194.

The control device is in signal connection with the encoder 111, and is configured to output an angle signal of the rotating and twisting actions to the slave-side execution apparatus of the surgical robot based on the rotation angle of the rotating shaft 120 sensed by the encoder 111, thereby realizing the effect of controlling the slave-side execution apparatus to perform the rotating and twisting actions by the doctor's operation of the rotation of the handle 130.

Referring to FIGS. 1 and 2 and FIGS. 5 to 7, the specific structure of the speed regulating device 150 is shown. The speed regulating device 150 is connected to the respective stators 138 of the two handles 130 to receive the axial movement of the handles 130. Specifically, the speed regulating device 150 includes two trigger assemblies 170 and two sensor assemblies 180 to correspond to the two handles 130, respectively. The two trigger assemblies 170 are respectively connected to the respective stators 138 of the two handles 130, such that the trigger assemblies 170 is capable of following the axial movement of the handle 130. The two sensor assemblies 180 are disposed on the base 161, and the positions of the two correspond to the positions of the two trigger assemblies 170 respectively, so as to sense the position of the trigger assemblies 170.

The control device is further in signal connection with the two sensor assemblies 180, and is configured to output a speed signal of the advancing action or the retracting action to the slave-side execution apparatus based on the movement amplitude of the trigger assemblies 170 following the handle 130 along the direction away from the baffle 121 sensed by the sensor assemblies 180. The speed magnitude is positively related to the movement amplitude.

The fixing frame 162 is provided with a guide part 163 extending in a direction parallel to the axial direction of the rotating shaft 120, and the guide part 163 is located below the rotating shaft 120. Specifically, the two guide parts 163 on the two fixing frames 162 extend toward each other. The trigger assembly 170 is movably disposed on the guide part 163. In another optional embodiment, such as the embodiment shown in FIGS. 8 and 9, a continuous guide part 163 may be provided between the two fixing frames 162.

Thus, the baffle 121 on the rotating shaft 120 limits one end point of the axial movement of the handle 130, which in turn limits one end point of the movement of the trigger assembly 170. The fixing frame 162 limits another end point of the movement of the trigger assembly 170. That is, the baffle 121 and the fixing frame 162 limit the trigger assembly 170 to be movable between a first position and a second position.

Wherein, the trigger assembly 170 is located at the end portion of the guide part 163 away from the fixing frame 162 when the handle 130 is in contact with the baffle 121. At this moment, the trigger assembly 170 is located at the first position. The trigger assembly 170 is located at the second position when the trigger assembly 170 is in contact with the fixing frame 162.

Moreover, when the trigger assembly 170 is in the first position, the control device is configured not to output the speed signal to the slave-side execution apparatus, or is configured to output a signal with a speed of zero. When the trigger assembly 170 is in the second position, the control device is configured to output a signal to the slave-side execution apparatus to advance or retract at a maximum speed.

The trigger assembly 170 includes a moving member 171, a triggering member 172 and a stator connecting member 173. Wherein, the moving member 171 is sleeved on the guide part 163 so as to be movable along the guide part 163. Optionally, the moving member 171 is configured as a linear bearing. The stator connecting member 173 is disposed at an end portion of the moving member 171 close to the fixing frame 162, and extends upward to connect with the stator 138 for transmitting the axial movement of the handle 130. The triggering member 172 is connected to the end portion of the moving member 171 away from the fixing frame 162 to interact with the sensor assembly 180.

Specifically, the sensor assembly 180 is configured to be able to sense a step change or linear change of the movement amplitude of the trigger assembly 170. The control device is configured to be able to output a step speed signal or a linear speed signal of the advancing action or the retracting action to the slave-side execution apparatus based on the step or linear change of the above-mentioned movement amplitude. Wherein, the speed magnitude of the step speed signal or the linear speed signal increases gradually in the direction of movement of the trigger assembly 170 toward the corresponding fixing frame 162.

Exemplarily, the sensor assembly 180 includes at least two photoelectric sensors 181 arranged side by side on the base 161 along the moving direction of the handle 130. Therefore, the control device is configured to output a step speed signal of the advancing motion or the retracting motion to the slave-side execution apparatus based on the step change of the movement amplitude of the trigger assembly 170 sensed by the sensor assembly 180. Optionally, the sensor assembly 180 includes three photoelectric sensors 181 corresponding to three speed steps of high, medium and low, respectively. Moreover, along the moving direction of the trigger assembly 170 approaching the fixing frame 162, the speeds of the speed signals corresponding to the three photoelectric sensors 181 increase sequentially, that is, first low, then middle, and then high. In this way, the speed of advancing or retracting can be controlled to ensure the safety of operation.

Optionally, the sensor assembly 180 may include more photoelectric sensors 181, such as four, five, six, seven, etc., to correspond to more speed steps control.

Wherein, the base 161 is provided with a mounting groove 165 passing through the base 161. The mounting groove 165 extends along the length direction of the base 161, or in other words, the mounting groove 165 extends along a direction parallel to the axial direction of the rotating shaft 120. More specifically, a mounting block 166 is disposed in the mounting groove 165 and also extends along the length direction of the base 161. The mounting block 166 is disposed at the inner sidewall of the mounting groove 165, and the upper surface of the mounting block 166 is lower than that of the base 161 to form a stepped structure. Optionally, two mounting blocks 166 are respectively disposed at two inner sidewalls of the mounting groove 165. More preferably, the lower surface of the mounting block 166 is flush with that of the base 161.

The photoelectric sensor 181 is installed in the mounting groove 165 and forms a snap-fit with the above-mentioned stepped structure. In other words, a part of the photoelectric sensor 181 is located in the middle of the mounting groove 165 to be suspended, and the other part is overlapped on the mounting block 166 to form a fixed connection.

The top of the photoelectric sensor 181 is optionally provided with recesses, and the respective recesses of the three photoelectric sensors 181 have the same height and can be sequentially abutted or corresponded to form a sensing channel 182. The triggering member 172 is bent and extends toward the sensor assembly 180, such that when the moving member 171 is moved along the guide part 163, the triggering member 172 can enter the sensing channel 182, thereby realizing the interaction with the photoelectric sensor 181.

Optionally, the distance among the three photoelectric sensors 181 can be adjusted to divide the travel of the handle 130 into three intervals, corresponding to three speed steps of high, medium, and low, respectively. For example, the low-speed interval can be made longer, the medium-speed interval is the second, and the high-speed interval is the shortest, so as to ensure the safety of the operation.

As another implementation, the sensor assembly 180 may be a displacement sensor, an optical grating sensor, a magnetic grating sensor, or the like. Therefore, the control device is further configured to output a linear speed signal of the advancing action or the retracting action to the slave-side execution apparatus based on the linear change of the movement amplitude of the trigger assembly 170 sensed by the sensor assembly 180. Moreover, along the moving direction of the trigger assembly 170 approaching the fixing frame 162, the speed of the linear speed signal increases linearly. Thus, the stepless speed change of the advancing action or retracting action of the slave-side execution apparatus can be achieved.

Exemplarily, referring to FIGS. 8 and 9, the sensor assembly is configured as a magnetic grating sensor 285 fixed on the base 161 through a fixing seat 284. The trigger assembly includes a magnetic strip 286. The moving member has a mounting part 274 extending along the extension direction of the guiding part. The magnetic strip 286 is disposed on the mounting part 274 such that the magnetic strip 286 is provided to extend along the axial direction of the rotating shaft 120. In the illustrated embodiment, the magnetic strip 286 is located on the side of the magnetic grating sensor 285. Preferably, the magnetic strip 286 is spaced apart from the magnetic grating sensor 285, and the distance between the magnetic strip and the magnetic grating sensor 285 is preferably 0.1 to 0.5 mm. More preferably, the distance between the magnetic strip 286 and the magnetic grating sensor 285 is 0.3 mm.

The master-side control mechanism 100 based on the present disclosure is capable of simultaneously simulating the pushing, pulling, rotating and twisting actions of the guidewire or catheter, and the two handles 130 are disposed relatively independently, so that a non-interfering interlocking between the advancing action and the retracting action can be realized while the operation feel of pinching the guidewire or catheter and the rotating operation feel are simulated, and the speed of the pushing and pulling actions can be adjusted.

The aforementioned mode in which the displacement of the handle 130 corresponds to the advancing or retracting speed of the guidewire or catheter can be referred to as a first mode of the control device. In addition, the control device in the present disclosure may also be configured with a second mode and/or a third mode in other embodiments, wherein the second mode is a mode in which the displacement of the handle 130 corresponds to an advancing or retracting displacement of the guidewire or catheter, and the third mode is a mode in which the advancing or retracting speed and advancing or retracting displacement of the guidewire or catheter are controlled by an operation button 275. Wherein, the control device may be configured with one or two of the first mode, the second mode and the third mode, and may also be configured with all the above three modes for the doctor to freely select.

In another optional embodiment, the sensor assembly may be a sensing device capable of sensing a linear change of the movement amplitude of the trigger assembly 170, such as a displacement sensor, an optical grating sensor or a magnetic grating sensor 285.

On this basis, the control device is configured with a second mode, in which the control device is configured to output a movement displacement signal of the advancing action or the retracting action to the slave-side execution apparatus based on the movement amplitude, and the magnitude of the movement displacement corresponds to the movement amplitude.

Exemplarily, the handle 130 can be moved 50 mm at most in the axial direction. In the second mode, when the handle 130 corresponding to the advancing action is moved, the guidewire or catheter of the slave-side execution apparatus can follow the handle 130 to advance in real time, and the advancing speed is preferably consistent with the moving speed of the handle 130. When the handle 130 is moved to the maximum displacement but the guidewire or catheter is still not advanced in place, the doctor can release the handle 130 to allow it to reset naturally, and move the handle 130 again to advance the guidewire or catheter a further 50 mm. Thus, the guidewire or catheter can be continuously advanced by advancing and resetting the handle 130. The control mode of the other handle 130 corresponding to the retracting action is similar to the control mode of the handle 130 corresponding to the advancing action described above, which will not be repeated here.

The third mode of the control device can be selected if a more precise operation is needed by the doctor. Continuing to refer to FIG. 8, the handle 130 is provided with an operation button 275. The respective operation buttons 275 on the two handles 130 correspond to the advancing action and the retracting action, respectively. An end portion of the handle 130 is also provided with an end cover 239 connected with the stator 138. The operation button 275 is arranged at the joint of the end cover 239 and the stator 138.

In the third mode, the control device is configured to control the slave-side execution apparatus to perform a predetermined displacement of the advancing action or to perform a predetermined displacement of the retracting action when the operation button 275 is pressed for no more than a first predetermined duration. The slave-side execution apparatus is controlled to perform the advancing action or the retracting action at a preset speed when the operation button 275 is pressed for more than a second predetermined duration, and the advancing action or the retracting action is controlled to stop when the operation button 275 is reset, wherein the second predetermined duration is longer than or equal to the first predetermined duration.

Exemplarily, the guidewire or catheter may be controlled to advance or retract by 1 mm for each short press of the operation button 275. The guidewire or catheter may be controlled to advance or retract at a low and uniform speed when the operation button 275 is pressed for a long time. The guidewire or catheter can be stopped immediately when the operation button 275 is released.

Exemplarily, the first predetermined duration may be 0.1 to 1 s. When the operation button 275 is pressed for no more than 1 s, the current operation is considered as a short press. The second predetermined duration may be 1 to 2 s. When the operation button 275 is pressed for more than 1 s, the current operation may be considered as a long press.

Therefore, the control scheme of the third mode can prevent the guidewire or catheter from being "overfed" or "underfed" due to the trembling of the doctor's hands.

Unless otherwise defined, the technical and scientific terms used herein have the same meanings as commonly understood by those skilled in the art of the present disclosure. The terms used herein are only for describing specific implementation purposes, and are not intended to limit the present disclosure. A feature described in one embodiment herein can be applied to another embodiment alone or in combination with other features, unless the feature is not applicable in the other embodiment or otherwise stated.

The present disclosure has been described through the above-mentioned embodiments, but it should be understood that the above-mentioned embodiments are only for the purpose of illustrations and descriptions, and are not intended to limit the present disclosure to the scope of the described embodiments. Furthermore, those skilled in the art can understand that the present disclosure is not limited to the above embodiments. More variations and modifications can be made based on the enlightenment of the present disclosure, and these variations and modifications fall within the protection scope of the present disclosure as defined in the appended claims and their equivalents.

## Claims

1. A master-side control mechanism for a surgical robot, comprising:
a base;
two fixing frames disposed opposite to each other at both ends of the base;
a rotating shaft rotatably connected to the two fixing frames and provided with a baffle in the middle;
a first sensing device configured to sense a rotational movement of the rotating shaft;
two handles respectively located on both sides of the baffle and sleeved on the rotating shaft, the two handles being configured to be capable of moving opposite to each other relative to the rotating shaft along an axial direction of the rotating shaft;
two second sensing devices configured to respectively sense an axial movement of the two handles relative to the rotating shaft; and
a control device in signal connection with the first sensing device and the two second sensing devices;
wherein the control device is configured to control a slave-side execution apparatus of the surgical robot to perform rotating and twisting actions based on the rotational movement of the rotating shaft sensed by the first sensing device; and
the axial movement of the two handles corresponds respectively to an advancing action and a retracting action of the slave-side execution apparatus, and the control device is configured to control the slave-side execution apparatus to perform the advancing action or the retracting action based on the axial movement of the two handles sensed by the two second sensing devices.

2. The master-side control mechanism of claim 1, wherein,
the first sensing device is configured as an encoder arranged at an end portion of the rotating shaft and used to sense a rotation angle of the rotating shaft; and
the control device is configured to output an angle signal of the rotating and twisting actions to the slave-side execution apparatus of the surgical robot based on the rotation angle of the rotating shaft sensed by the encoder.

3. The master-side control mechanism of claim 2, wherein,
the handle comprises an operating body capable of co-rotating with the rotating shaft and a non-rotating stator;
the master-side control mechanism further comprises two trigger assemblies respectively connected to the respective stators of the two handles, such that the trigger assemblies are capable of following the axial movement of the handles; and
the two second sensing devices are configured as two sensor assemblies arranged on the base and corresponding respectively to the two trigger assemblies to sense the positions of the trigger assemblies.

4. The master-side control mechanism of claim 3, wherein the two second sensing devices are configured to sense a movement amplitude of the trigger assemblies in a direction away from the baffle; and
the control device is configured with a first mode in which the control device is configured to output a speed signal of the advancing action or the retracting action to the slave-side execution apparatus based on the movement amplitude and the speed magnitude is positively related to the movement amplitude.

5. The master-side control mechanism of claim 4, wherein,
the baffle and the fixing frame are configured to limit the trigger assembly to be movable between a first position and a second position;
wherein the trigger assembly is located at the first position when the handle is in contact with the baffle, and the trigger assembly is located at the second position when the trigger assembly or the handle is in contact with the fixing frame;
when the trigger assembly is located at the first position, the control device is configured not to output the speed signal to the slave-side execution apparatus, or is configured to output a signal with a speed of zero; and
when the trigger assembly is located at the second position, the control device is configured to output a signal to the slave-side execution apparatus to advance or retract at a maximum speed.

6. The master-side control mechanism of claim 5, wherein the sensor assembly comprises at least two photoelectric sensors arranged side by side along a moving direction of the handle, such that the sensor assembly is capable of sensing a step change in the movement amplitude of the trigger assembly; and
the control device is configured to output a step speed signal of the advancing action or the retracting action to the slave-side execution apparatus based on the step change sensed by the sensor assembly, each of the photoelectric sensors corresponds to a step speed signal, and the speed magnitudes of the speed signals corresponding to the at least two photoelectric sensors are increased sequentially along the moving direction of the trigger assembly approaching the fixing frame.

7. The master-side control mechanism of claim 5, wherein the sensor assembly comprises a displacement sensor or an optical grating sensor, such that the sensor assembly is capable of sensing a linear change of the movement amplitude of the trigger assembly; and
the control device is configured to output a linear speed signal of the advancing action or the retracting action to the slave-side execution apparatus based on the linear change sensed by the sensor assembly, and the speed magnitude of the linear speed signal is increased linearly along the moving direction of the fixing frame approaching the trigger assembly.

8. The master-side control mechanism of claim 3, wherein,
the two sensor assemblies are configured to sense movement amplitudes of the trigger assemblies in a direction away from the baffle; and
the control device is configured with a second mode, in which the control device is configured to output a movement displacement signal of the advancing action or the retracting action to the slave-side execution apparatus based on the movement amplitude, and a magnitude of the movement displacement corresponds to the movement amplitude.

9. The master-side control mechanism of claim 8, wherein,
the sensor assembly comprises a magnetic grating sensor fixed on the base through a fixing seat; and
the trigger assembly comprises a magnetic strip provided to extend along the axial direction of the rotating shaft and spaced apart from the magnetic grating sensor.

10. The master-side control mechanism of claim 3, wherein,
the handle is provided with an operation button, and the respective operation buttons on the two handles correspond to the advancing action and the retracting action, respectively; and
the control device is configured with a third mode, in which the control device is configured to control the slave-side execution apparatus to perform a predetermined displacement of the advancing action or to perform a predetermined displacement of the retracting action when the operation button is pressed for no more than a first predetermined duration.

11. The master-side control mechanism of claim 10, wherein in the third mode, the control device is further configured to:
control the slave-side execution apparatus to perform the advancing action or the retracting action at a preset speed when the operation button is pressed for more than a second predetermined duration, and control the advancing action or the retracting action to stop when the operation button is reset; and
wherein the second predetermined duration is longer than or equal to the first predetermined duration.

12. The master-side control mechanism of claim 10, wherein an end portion of the handle is also provided with an end cover connected with the stator, and the operation button is arranged at the joint of the end cover and the stator.

13. The master-side control mechanism of any one of claims 5 to 12, wherein the master-side control mechanism further comprises a guide part connected to the fixing frame and located below the rotating shaft and extending in a direction parallel to the axial direction of the rotating shaft, and the trigger assembly is movably arranged on the guide part.

14. The master-side control mechanism of claim 13, wherein the trigger assembly comprises:
a stator connecting member connected to the stator;
a moving member connected with the stator connecting member and sleeved on the guide part, and being movable along an extension direction of the guide part; and
a triggering member connected to the moving member.

15. The master-side control mechanism of claim 14, wherein,
the sensor assembly comprises at least two photoelectric sensors, the tops of the photoelectric sensors are provided with recesses, and the respective recesses of the at least two photoelectric sensors are sequentially abutted or corresponded to form a sensing channel; and
the triggering member is bent and extends toward the sensor assembly, such that the triggering member can enter the sensing channel when the moving member moves along the guide part.

16. The master-side control mechanism of claim 14, wherein the moving member has a mounting part extending along the extension direction of the guide part, and the triggering member is configured as a magnetic strip arranged on the mounting part.

17. The master-side control mechanism of claim 16, wherein the sensor assembly comprises a magnetic grating sensor, the magnetic strip is located on the side of the magnetic grating sensor, and a distance between the magnetic strip and the magnetic grating sensor is 0.1 to 0.5 mm.

18. The master-side control mechanism of any one of claims 3 to 12, wherein the operating body is sleeved on the rotating shaft, the operating body and the rotating shaft are configured as a profile-fit, the stator is arranged at an end portion of the operating body away from the baffle, and the handle further comprises a first bearing having an outer ring connected to the operating body of the handle and an inner ring connected to the stator.

19. The master-side control mechanism of claim 18, wherein the rotating shaft is provided with a blocking portion protruding from the rotating shaft, the handle further comprises a conforming portion located within and fixedly connected with the operating body and configured to be sleeved on and profile-fitted with the rotating shaft, and an elastic member fixedly connected between the blocking portion and the conforming portion and configured to provide an elastic force to the handle such that the handle tends to move in a direction close to the baffle.

20. The master-side control mechanism of claim 19, wherein a cross-section of the rotating shaft is configured as a rectangle, the conforming portion is provided with a through hole adapted to the rectangle, and the rotating shaft passes through the operating body via the through hole.

21. The master-side control mechanism of claim 19, wherein,
the rotating shaft is provided with a spline extending along the axial direction; and
the conforming portion is configured as a ball spline pair which is configured to be sleeved on the rotating shaft and adapted to the spline, such that the operating body is capable of co-rotating with the rotating shaft and moving along an extension direction of the spline.

22. The master-side control mechanism of any one of claims 1 to 12, wherein the top of the fixing frame is further provided with a second bearing having an outer ring fixedly connected with the fixing frame and an inner ring fixedly connected with an end portion of the rotating shaft.

23. The master-side control mechanism of claim 22, wherein the master-side control mechanism further comprises a damping assembly arranged at the end portion of the rotating shaft to adjust the resistance when the rotating shaft rotates.

24. The master-side control mechanism of claim 23, wherein the rotating shaft passes through the fixing frame;
the damping assembly comprises a friction plate, a stop washer, a spring washer and a locknut arranged in sequence from the fixing frame in a direction away from the fixing frame; or
the damping assembly comprises a damping ring sleeved on the rotating shaft and a locknut sleeved on the rotating shaft and connected to a side of the fixing frame.

25. A surgical robot, comprising the master-side control mechanism of any one of claims 1 to 24.
